# EUROPEAN PATENT APPLICATION

(11) **EP 4 306 143 A1**
(43) Date of publication of application: **17.01.2024**
(21) Application number: 22184075.4
(22) Date of filing: 11.07.2022
(51) Int. Cl.: A61M 1/06

(54) **MILK EXPRESSION SYSTEM AND METHOD**

(71) Applicant: Koninklijke Philips N.V., 5656 AG Eindhoven (NL)
(72) Inventor: WADHWA, Sahil, Eindhoven (NL); CLAASSEN, Coen Petrus Martinus, Eindhoven (NL); VAN VELDHUIZEN, Gijsbert Hendrik, Eindhoven (NL); DOBRUSSKIN, Christoph, Eindhoven (NL)
(74) Representative: Philips Intellectual Property & Standards

(57) **Abstract**

Provided is a milk expression system (1). The system comprises a first pump arrangement (8A) configured to operate according to a first modality comprising a first expression operation and a first sensory feedback operation. The system further comprises a second pump arrangement (8B) configured to operate according to a second modality comprising a second expression operation. The first pump arrangement may thus be employed in milk expression from one of a user's breasts while the second pump arrangement is employed in milk expression from the other of the user's breasts. The first and second pump arrangements are configured to cooperate to coordinate implementation of the first and second modalities so that sensory feedback provided by the first sensory feedback operation is easier to discern and/or interpret.

## Description

### FIELD OF THE INVENTION

This invention relates to milk expression systems, in particular with a breast pump arrangement for each breast. The invention further relates to a method for operating such a milk expression system.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted.

For example, a breast pump applies a baseline vacuum. One mode of operation can comprise repeating vacuum strokes, e.g. a first mode of repeating strokes for stimulation and a second mode for extraction. A baseline vacuum can be added on top of a varying vacuum to keep the cup in position on the breast. During the stimulation mode, the milk ejection reflex is stimulated. When the breast pump is activated, a pressure source such as a vacuum pump inside the breast pump generates a vacuum and the stimulation mode can be started. The extraction mode follows the stimulation mode. The vacuum pump is typically driven by an electric motor. For portable breast pumps, these motors are driven by battery power.

Wearable breast pump arrangements are characterized in that they can be fully worn on the user's body, allowing the user to move around freely. Some of these are small enough to fit into a user's bra. A motor, battery and milk container are for example integrated to form a single unit.

Other combinations are known, such as semi-wearable in which expression kits are body worn and a driving power unit is not neccearily body-worn, e.g. not being configured to interface with the body.

It is known to have some kind of interface to provide feedback to the user, such as a tactile or audible feedback when control buttons are pressed, or feedback for notifying the user that device the expression cycle is finished, or for an empty battery alert etc. This user feedback is traditionally delivered by means of sound, light, haptic feedback, mobile phone notification or other methods.

Wearable breast pumps may be used in public places, and being unobtrusive is in those situations preferred. Hence haptic feedback is a preferred way of giving feedback in such situations.

Often, it is desirable to express both breasts at the same time. A user will then typically acquire a set of identical devices to achieve a setup which performs stimulation and expression independently for each breast.

Alternatively, a single system enabling dual expression, in other words expression from both breasts at the same time, may be used, such as the Philips Avent SCF398/11.

When using two breast pumps at the same time, the noise and suction of the two devices is very likely to be not in sync resulting in a non-uniform sound, mismatch in cycle sounds and mismatch in suction feeling at both breasts. Such a scenario may further risk that any feedback, such as tactile or audible feedback, provided to the user from one of the standalone devices is more difficult to discern and interpret because of the additional sensory influence, e.g. noise, associated with operation of the other device. In other words, the tactile or audible feedback can be "masked" by this additional sensory influence, e.g. noise, from the other device.

### SUMMARY OF THE INVENTION

The invention is defined by the claims.

According to examples in accordance with an aspect of the invention, there is provided a milk expression system comprising: a first pump arrangement configured to operate according to a first modality comprising a first expression operation and a first sensory feedback operation; and a second pump arrangement configured to operate according to a second modality comprising a second expression operation, wherein the first and second pump arrangements are configured to cooperate to coordinate implementation of the first and second modalities.

The milk expression system may comprise a first expression kit comprising the first pump arrangement, and a second expression kit comprising the second pump arrangement.

In some embodiments, at least one of the first expression kit and the second expression kit is a wearable expression kit.

In some embodiments, the first expression kit comprises, in addition to the first pump arrangement, a first funnel, in other words a first breast shield, for receiving one of the mother's breasts.

Alternatively of additionally, the second expression kit may comprise, in addition to the second pump arrangement, a second funnel, in other words a second breast shield, for receiving one of the mother's breasts.

The first sensory feedback operation may provide information to the user relating to operation of the first pump arrangement, for example information about the status of the milk expression session, or confirmatory feedback in response to input commands provided by the user.

The first sensory feedback operation may, for example, be provided during times of interruption of the first expression operation of the first pump arrangement if sensory feedback is needed is during breast pumping, or it may be before the start of the first expression operation or after the end of the first expression operation.

As long as the sensory feedback provided by the first sensory feedback operation differs from a sensory contribution provided by the first expression operation (which first expression operation may include a stimulation mode and an extraction mode), it can provide information content to the user.

The first pump arrangement may be configured to implement the first sensory feedback operation for one or more of the following: a user has pressed an input button of the first pump arrangement; a battery of the first pump arrangement is at or approaching depletion; a milk container into which milk is being expressed is full; a remaining time for an expression cycle or other time indication; an expression cycle has ended; a state of readiness; a malfunction; and a leak being detected.

By the first and second pump arrangements cooperating to coordinate implementation of the first and second modalities, the sensory feedback provided by the first sensory feedback operation can be more easily distinguished by the user from the sensory output caused by operation of the second pump arrangement according to the second modality. Thus, the sensory feedback provided by the first sensory feedback operation may be easier to discern and/or interpret, in spite of the additional sensory influence associated with operation of the second pump arrangement.

It is noted that the term "expression operation" as used herein refers to operation of the respective pump arrangement to express milk from the breast, and optionally to prepare the breast for milk expression.

Operation of the respective pump arrangement to express milk from the breast may be implemented in an extraction mode.

In some embodiments, preparing the breast for milk expression is implemented via one or more of a massage mode and a stimulation mode included in the expression operation.

Alternatively or additionally, preparing the breast for milk expression may be via a positioning mode included in the expression operation. The positioning mode prepares the breast for expression by the respective pump arrangement providing an under-pressure to the user to position a funnel/breastshield over their breast while observing the correct alignment.

In some embodiments, a first controller is configured to control the first pump arrangement according to the first modality, and a second pump controller is configured to control the second pump arrangement according to the second modality.

In such embodiments, the second controller may be configured to send second system data relating to the second modality to the first controller, with the first controller being configured to select control settings associated with the first modality based on the second system data to coordinate the first and second modalities.

Alternatively or additionally, the first controller may be configured to send first system data relating to the first modality to the second controller, with the second controller being configured to select control settings associated with the second modality based on the first system data to coordinate the first and second modalities.

The first and second controllers may be connected to each other in any suitable manner in order to cooperate in the described manner, for example the system may include a wired or wireless communication link connecting the first controller and the second controller to each other.

It is noted that the first and second controllers can be part of the same physical hardware and potentially operate and/or behave as a single controller.

In some embodiments, said coordinated implementation of the first and second modalities comprises the first sensory feedback operation being provided while the second expression operation of the second pump arrangement is paused.

By pausing operation of the second pump arrangement in this manner, the concomitant temporary cessation of the sensory output caused by the second expression operation of the second pump arrangement may make the sensory feedback provided by the first sensory feedback operation more easily discernible.

As an alternative to such temporary cessation, attenuation of the sensory output caused by the second expression operation of the second pump arrangement, reduction in frequency of a signal of the sensory output, etc. can be used.

In some embodiments, the second modality comprises the second expression operation and a second sensory feedback operation.

The second sensory feedback operation may provide information to the user relating to operation of the second pump arrangement, for example information about the status of the milk expression session, or confirmatory feedback in response to input commands provided by the user.

The second sensory feedback operation may, for example, be provided during times of interruption of the second expression operation of the second pump arrangement if sensory feedback is needed is during breast pumping, or it may be before the start of the second expression operation or after the end of the second expression operation.

The second pump arrangement may be configured to implement the second sensory feedback operation for one or more of the following: a user has pressed an input button of the second pump arrangement; a battery of the second pump arrangement is at or approaching depletion; a milk container into which milk is being expressed is full; a remaining time for an expression cycle or other time indication; an expression cycle has ended; a state of readiness; a malfunction; and a leak being detected.

As long as the sensory feedback provided by the second sensory feedback operation differs from a sensory contribution provided by the second expression operation (which second expression operation may include a stimulation mode and an extraction mode), it can provide information content to the user.

The coordination of the first and second modalities may nonetheless mean that the sensory feedback provided by the first sensory feedback operation of the first pump arrangement can be more easily distinguished by the user from the sensory output provided by one or both of the second expression operation and the second sensory feedback operation.

In some embodiments, said coordinated implementation of the first and second modalities comprises sensory feedback provided by the first sensory feedback operation being different from sensory feedback provided by the second sensory feedback operation.

The different sensory feedback provided by the first sensory feedback operation compared to that provided by the second sensory feedback operation may provide a relatively simple way of assisting the user to distinguish the feedbacks provided by the first and second pump arrangements.

In some embodiments, said coordinated implementation of the first and second modalities comprises sensory feedback provided by the first sensory feedback operation having a different intensity, duration, and/or number of repetitions in a feedback signal relative to sensory feedback provided by the second sensory feedback operation.

Patterns, rhythms, ramp up/down may alternatively or additionally distinguish the sensory feedback provided by the first sensory feedback operation relative to the sensory feedback provided by the second sensory feedback operation.

The term "different intensity" may mean that the sensory feedback provided by the first sensory feedback operation has a different intensity level from the intensity level of the sensory feedback provided by the second sensory feedback operation.

In some embodiments, said coordinated implementation of the first and second modalities comprises the first sensory feedback operation being asynchronous with the second expression operation.

By the first sensory feedback operation being implemented at a different time from the second expression operation of the second pump arrangement, the sensory feedback provided by the former may be more easily differentiated from the contribution to the sensory output provided by the latter.

In some embodiments, said coordinated implementation of the first and second modalities comprises the first sensory feedback operation being concurrent with the second sensory feedback operation.

This may mean that the second sensory feedback operation may be asynchronous with the first expression operation. Thus, the sensory feedback provided by the second sensory feedback operation of the second pump arrangement may be differentiated from a sensory contribution provided by the first expression operation of the first pump arrangement.

In some embodiments, the first sensory feedback operation may be concurrent with, and provide identical sensory feedback to that provided by, the second sensory feedback operation.

In such embodiments, the concurrent sensory feedback provided by the first and second sensory feedback operations may mean that the sensory feedback is emphasized.

Cooperation of the first and second pump arrangements in order to coordinate implementation of the first and second modalities can be achieved in various ways.

In some embodiments, the first and second pump arrangements are controllable by one or more controllers, e.g. running a suitable pump arrangement control algorithm, to coordinate the first and second modalities.

The above-mentioned first and second controllers may have identical communication functionality. Alternatively, one of the first and second controllers may be a master controller with the other of the first and second controllers being a slave controller.

In embodiments in which one of the first and second controllers is a master controller and the other of the first and second controllers is a slave controller, communication may only be needed from the master controller to the slave controller.

The milk expression system may comprise a communications system configured to enable each of the first and second controllers to communicate with an external controller device configured to control the first and second controllers to cooperate to coordinate implementation of the first and second modalities.

Such an external controller device may be, for example, a smartphone or a tablet computer.

Communication between the external controller device and the first and second controllers may be implemented in any suitable manner, such as via wireless communication, e.g. via sound, light or any other type of wireless communication link, or via wired communication.

In alternative embodiments, the first and second controllers may be configured to cooperate with no intermediary.

The sensory feedback provided by the first sensory feedback operation may be one or more of audible, optical, and haptic feedback.

For example, the first breast pump arrangement may include one or more light emitters, and the first sensory feedback operation comprises controlling the optical output of the one or more light emitters to provide the sensory feedback to the user.

Alternatively or additionally, the first breast pump arrangement may include one or more loudspeakers, and the first sensory feedback operation comprises controlling the one or more loudspeakers to provide the sensory feedback to the user.

In some embodiments, the sensory feedback is visual, tactile and/or audible feedback.

More generally, the first pump arrangement may include a first drive arrangement that implements the first expression operation by applying and releasing pressure.

The pressure may be generated as an air pressure applied to the breast or applied to a membrane, or it may be generated as a hydraulic pressure applied to a membrane (which then creates an air pressure in a cavity around the nipple and a portion of the breast) or it may be generated as a mechanical pressure applied to the breast or to such a membrane. The pressure may be any pressure which, once the milk flow has been initiated, uses the milk in a cavity around the nipple and a portion of the breast to create a partial or fully hydraulic pressure in said cavity.

The first drive arrangement may thus be regarded as those parts of the first pump arrangement which are used to create a pressure profile for application to the breast (directly or indirectly).

The applying and releasing pressure may also be what is used, albeit in a different way, when the first sensory feedback operation is implemented to provide the sensory feedback.

Thus, in some embodiments, the drive arrangement is configured to implement the first sensory feedback operation.

Tactile/haptic feedback provided via the drive arrangement may be a type of feedback particularly susceptible to "dissolve" in the sensory output caused by operation of the second pump arrangement, e.g. pumping interactions, since it may use the same principles, e.g. touch and/or suction and/or nipple stretching. This may be alleviated by the coordination of implementation of the first and second modalites described herein.

Controlling the first pump arrangement to implement the first sensory feedback operation may involve controlling the first drive arrangement to generate a particular pressure waveform different from that generated when implementing the first expression operation, thereby to provide an audible and/or tactile feedback signal.

In such embodiments, the pressure waveform generated in the implementation of the first sensory feedback operation is different from each mode included in the first expression operation so that the first sensory feedback operation provides information content to the user.

Thus, in embodiments in which the first expression operation includes one or more of the stimulation mode, the extraction mode, the positioning mode and the massage mode, the pressure waveform generated in the implementation of the first sensory feedback operation is different from each said mode.

At least one of the first and second pump arrangements may be a wearable pump arrangement.

Being assisted, via coordination of the first and second modalities, to distinguish the sensory feedback provided by the first feedback operation of the first pump arrangement from the sensory output of the second pump arrangement may be particularly valuable when one or both of the first and second pump arrangements are being worn by the user.

According to another aspect there is provided a method for operating a milk expression system having a first pump arrangement and a second pump arrangement, the method comprising: controlling the first pump arrangement according to a first modality comprising a first expression operation and a first sensory feedback operation; and controlling the second pump arrangement according to a second modality, wherein the controlling the first pump arrangement and the controlling the second pump arrangement are cooperative to coordinate implementation of the first and second modalities.

The method may be for the non-therapeutic expression of milk.

Further provided is a computer program product comprising computer program code which is configured, when said computer program is run on one or more processors included in a milk expression system further comprising a first pump arrangement and a second pump arrangement, to cause the one or more processors to implement the method according to any of the embodiments disclosed herein.

Embodiments described in respect of the method or computer program product may be applicable to the system, and embodiments described in respect of the system may be applicable to the method or computer program product.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 shows a milk expression system according to an example;
FIG. 2 shows a mother wearing two wearable pumps arrangements;
FIG. 3 shows a milk expression system according to another example;
FIG. 4 shows a milk expression system according to a further example; and
FIG. 5 shows some feedback profiles that may be created as feedback signals.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a milk expression system. The system comprises a first pump arrangement configured to operate according to a first modality comprising a first expression operation and a first sensory feedback operation. The system further comprises a second pump arrangement configured to operate according to a second modality comprising a second expression operation. The first pump arrangement may thus be employed in milk expression from one of a user's breasts while the second pump arrangement is employed in milk expression from the other of the user's breasts. The first and second pump arrangements are configured to cooperate to coordinate implementation of the first and second modalities so that sensory feedback provided by the first sensory feedback operation is easier to discern and/or interpret.

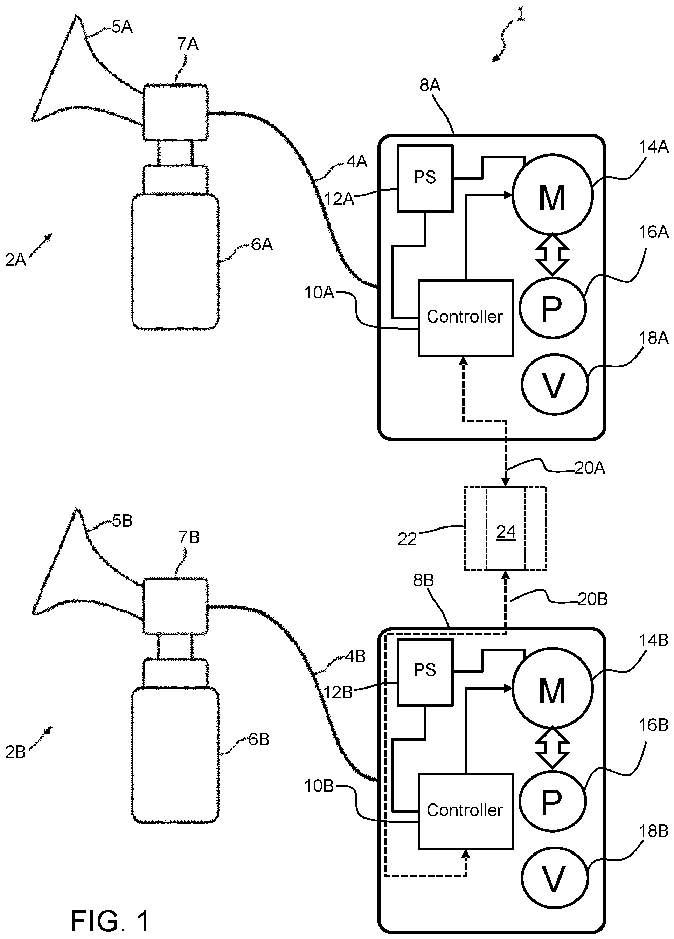

FIG. 1 shows a milk expression system 1, comprising a first expression kit 2A and a first pump arrangement 8A. The system 1 also includes a second expression kit 2B and a second pump arrangement 8B.

The first expression kit 2A comprises a main body 7A, a funnel 5A for receiving one of the mother's breasts, and a receptacle 6A for collecting the expressed milk.

In some embodiments, the first pump arrangement 8A is included in the first expression kit 2A. Alternatively, the first pump arrangement 8A and the first expression kit 2A may be supplied separately from each other.

The funnel 5A and the receptacle 6A are connected to the main body 7A. The main body 7A comprises a vacuum chamber. A flexible membrane or diaphragm is located in the vacuum chamber. The membrane prevents expressed milk from flowing into the tube 4A leading to the first pump arrangement 8A.

The first pump arrangement 8A may be controlled by a first controller 10A. In the nonlimiting example shown in FIG. 1, the first controller 10A is included in the first pump arrangement 8A, although it is also conceivable that the first controller 10A is a separate component from the first pump arrangement 8A.

The first pump arrangement 8A comprises a power source 12A, a motor 14A and a vacuum pump 16A. The first controller 10A controls the operation of the power source 12A, and motor 14A to drive the vacuum pump 16A. The pump arrangement 8A further comprises a solenoid valve 18A. In some embodiments, such as, for instance, in the case of a wearable pump arrangement, the power source 12A is provided separately from the pump arrangement.

In use, the vacuum pump 16A applies a vacuum to the membrane located in the main body 7A so that it deforms. The membrane deforms to create a vacuum in the funnel 5A which in turns applies a vacuum to the breast which enables milk to be expressed. Whilst this membrane may mean that the vacuum is applied indirectly to the breast, it should be understood that in an alternative embodiment, the vacuum is applied directly to the breast of a user. In this case, no such membrane may be included and the vacuum created by the vacuum pump is applied directly to the breast.

The vacuum is applied to the breast at intervals. That is, a pressure differential is applied on a cyclic basis. After a vacuum has been established, the pressure from the vacuum is released by the use of the solenoid valve 18A which is temporarily opened. The solenoid valve 18A is an electromechanically operated valve configured to open and close an air passage that connects to the vacuum side of the vacuum pump 16A to ambient air such that when the solenoid valve 18A is closed, the vacuum pump 16A generates a vacuum in the first expression kit 2A which enables milk to be expressed from the breast of a user. When the solenoid valve 18A is opened, the vacuum generated by the vacuum pump 16A is released as ambient air flows towards the vacuum or negative pressure created by the vacuum pump 16A such that the pressure exerted on the breast of a user is partially or completely reduced.

The second expression kit 2B comprises a main body 7B, a funnel 5B for receiving one of the mother's breasts, and a receptacle 6B for collecting the expressed milk. The design of the second expression kit 2B shown in FIG. 1 is the same as that of the first expression kit 2A described above, hence repetition of this description will be omitted.

It is nonetheless noted, that the first and second expression kits 2A, 2B need not have the same design as each other, such that embodiments in which the design of the first expression kit 2A is different from that of the second expression kit 2B can be contemplated.

In some embodiments, the second pump arrangement 8B is included in the second expression kit 2B. Alternatively, the second pump arrangement 8B and the second expression kit 2B may be supplied separately from each other.

In some embodiments, one or both of the first and second expression kits 2A, 2B comprise or comprises a milk sensor for sensing milk flow and for measuring flow volume.

Alternatively or additionally, different pressure waveforms may be selectable by a user, for example different intensity levels, depending on the level of comfort of the mother to those different intensity levels.

Different modes and settings have different cyclic frequency and different maximum under-pressure (i.e. vacuum) levels.

More generally, the first controller 10A may be configured to control the first pump arrangement 8A according to a first modality. Similarly, the second controller 10B may be configured to control the second pump arrangement 8B according to a second modality.

Whilst not shown in FIG. 1, it is noted that the first and second controllers 10A, 10B can be part of the same physical hardware and potentially operate and/or behave as a single controller.

The first and second modalities can, in some embodiments, comprise the same operations as each other. Hence it should be noted that the use of the terms "first" and "second" can serve merely to distinguish the modalities on the basis of them being implemented by the first controller 10A controlling the first pump arrangement 8A, or by the second controller 10B controlling the second pump arrangement 8B.

The first modality comprises a first expression operation and a first sensory feedback operation.

The first sensory feedback operation may provide information to the user relating to the operation of the first pump arrangement 8A, for example information about the status of the milk expression session, or confirmatory feedback in response to input commands provided by the user.

The first sensory feedback operation may, for example, be provided during times of interruption of the first expression operation of the first pump arrangement if sensory feedback is needed is during breast pumping, or it may be before the start of the first expression operation or after the end of the first expression operation.

As long as the sensory feedback provided by the first sensory feedback operation differs from a sensory contribution provided by the first expression operation, in particular each mode included in the first expression operation, it can provide information content to the user.

This sensory feedback provided by the first sensory feedback operation may be one or more of audible, optical, and haptic feedback.

For example, the first breast pump arrangement 8A may include one or more light emitters, and the first sensory feedback operation comprises controlling the optical output of the one or more light emitters to provide the sensory feedback to the user.

Alternatively or additionally, the first breast pump arrangement 8A may include one or more loudspeakers, and the first sensory feedback operation comprises controlling the one or more loudspeakers to provide the sensory feedback to the user.

The second modality comprises a second expression operation. The second expression operation can be the same as or different from the first expression operation.

The second modality may also include, in addition to the second expression operation, a second sensory feedback operation.

The second sensory feedback operation may provide information to the user relating to the operation of the second pump arrangement, for example information about the status of the milk expression session, or confirmatory feedback in response to input commands provided by the user.

The second sensory feedback operation may, for example, be provided during times of interruption of the second expression operation of the second pump arrangement if sensory feedback is needed is during breast pumping, or it may be before the start of the second expression operation or after the end of the second expression operation.

As long as the sensory feedback provided by the second sensory feedback operation differs from a sensory contribution provided by the second expression operation, in particular each mode included in the second expression operation, it can provide information content to the user.

This sensory feedback provided by the second sensory feedback operation may be one or more of audible, optical, and haptic feedback.

For example, the second breast pump arrangement 8B may include one or more light emitters, and the second sensory feedback operation comprises controlling the optical output of the one or more light emitters to provide the sensory feedback to the user.

Alternatively or additionally, the second breast pump arrangement 8B may include one or more loudspeakers, and the second sensory feedback operation comprises controlling the one or more loudspeakers to provide the sensory feedback to the user.

It is noted that the term "expression operation" as used herein refers to operation of the respective pump arrangement to express milk from the breast, and optionally to prepare the breast for milk expression.

Operation of the respective pump arrangement to express milk from the breast may be implemented in an extraction mode included in the respective expression operation.

In some embodiments, preparing the breast for milk expression is implemented via one or more of a massage mode and a stimulation mode included in the expression operation.

In the extraction mode, the vacuum can cycle between a baseline vacuum "Baseline_Vac" and a maximum vacuum (i.e. a maximum under-pressure) "Max_Vac".

In such embodiments, there is a first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline_Vac = atmospheric pressure (or in some cases lower than atmospheric pressure) Max_Vac = 300 mbar (30 kPa) below atmospheric pressure
TTV = 0.85 s
DI = 0.3 s
TTA = 0.05 s
DO = 0.35 s

This gives a total cycle time T_cycle of 1.55s.

These values are simply by way of example. The maximum under-pressure may be higher, for example 350 mbar (35 kPa) and the durations may differ. The cycle time is generally of the order of 1 to 2 seconds.

The stimulation mode for example has a faster frequency hence with a shorter cycle time of around 0.5 seconds, and a lower vacuum, for example a maximum under-pressure level of 150 mbar (15 kPa).

When the respective expression operation includes the stimulation mode and the extraction mode, the stimulation mode for example takes placing during an initial period of the breast pumping session (e.g. 50 seconds) and the extraction mode takes place during the remainder of the breast pumping session. There may also be different settings of extraction mode, again with different characteristics.

In some embodiments, the first expression operation of the first modality comprises a positioning mode, e.g. in addition to the stimulation mode and the extraction mode. Such a positioning mode may comprise intermittent application of a small under-pressure to allow the user to position the funnel 5A while observing the correct alignment.

In some embodiments, the first expression operation of the first modality comprises a massage mode, e.g. in addition to the stimulation mode and the extraction mode.

In some embodiments, the first sensory feedback operation of the first modality comprises the massage mode and the positioning mode, e.g. in addition to the stimulation mode and the extraction mode.

Turning to the second modality according to which the second pump arrangement 8B is controlled, this may comprise the second sensory feedback operation and the second expression operation, which second expression operation comprises such a positioning mode and/or massage mode, e.g. in addition to the stimulation mode and the extraction mode.

The motor 14A, pump 16A and/or valve 18A of the first pump arrangement 8A can be regarded as a first drive arrangement that, responsive to the first controller 10A, implements the first expression operation.

Similarly, the 14B, pump 16B and/or valve 18B of the second pump arrangement 8B can be regarded as a second drive arrangement that, responsive to the second controller 10B, implements the second expression operation.

The pressure may be generated as an air pressure applied to the breast or applied to the membrane, or it may be generated as a hydraulic pressure applied to a membrane (which then creates an air pressure in a cavity around the nipple and a portion of the breast) or it may be generated as a mechanical pressure applied to the breast or to the membrane. The pressure may be any pressure which, once the milk flow has been initiated, uses the milk in a cavity around the nipple and a portion of the breast to create a partial or fully hydraulic pressure in said cavity.

The first drive arrangement may thus, more generally, be regarded as those parts of the first pump arrangement 8A which are used to create a pressure profile for application to the breast (directly or indirectly).

Similarly, the second drive arrangement maybe regarded as those parts of the second pump arrangement 8B which are used to create a pressure profile for application to the breast (directly or indirectly).

The first expression operation may involve applying and releasing pressure, and this process of applying and/or releasing pressure may also be what is used, albeit in a different way, when the first sensory feedback operation is implemented to provide the sensory feedback.

Thus, in some embodiments, the first controller 10A is configured to control the first drive arrangement to implement the first sensory feedback operation.

Similarly, in some embodiments, the second controller 10B is configured to control the second drive arrangement to implement the second sensory feedback operation.

Controlling the first pump arrangement 8A to implement the first sensory feedback operation may involve controlling the first drive arrangement to generate a particular pressure waveform different from that generated when implementing the first expression operation thereby to provide an audible and/or tactile feedback signal.

In particular, the pressure waveform generated in the implementation of the first sensory feedback operation is different from each mode included in the first expression operation so that the first sensory feedback operation provides information content to the user.

Thus, in embodiments in which the first expression operation includes one or more of the stimulation mode, the extraction mode, the positioning mode and the massage mode, the pressure waveform generated in the implementation of the first sensory feedback operation is different from each said mode.

Similarly, the second expression operation may involve applying and releasing pressure, and this process of applying and/or releasing pressure may also be what is used, albeit in a different way, when the second sensory feedback operation is implemented to provide the sensory feedback.

Thus, in some embodiments, the second controller 10B is configured to control the second drive arrangement to implement the second sensory feedback operation.

Controlling the second pump arrangement 8B to implement the second sensory feedback operation may involve controlling the second drive arrangement to generate a particular pressure waveform different from that generated when implementing the second expression operation thereby to provide an audible and/or tactile feedback signal.

In particular, the pressure waveform generated in the implementation of the second sensory feedback operation is different from each mode included in the second expression operation so that the second sensory feedback operation provides information content to the user.

Thus, in embodiments in which the second expression operation includes one or more of the stimulation mode, the extraction mode, the positioning mode and the massage mode, the pressure waveform generated in the implementation of the second sensory feedback operation is different from each said mode.

The various modes and operations will result in different sounds generated by the pump arrangements 8A, 8B. In accordance with the invention, the coordinated control of the first and second pump arrangements 8A, 8B may assist the user, e.g. mother, to discern and/or interpret the additional user-recognizable sensory feedback (e.g. characteristic vibrations) provided by the first sensory operation included in the first modality according to which the first pump arrangement 8A is controlled.

Such coordination of implementation of the first and second modalities may take various forms.

In some embodiments, the coordinated implementation of the first and second modalities comprises the first sensory feedback operation being provided while operation of the second pump arrangement 8B is paused. By pausing operation of the second pump arrangement 8B in this manner, the concomitant temporary cessation of the sensory output caused by operation of the second pump arrangement 8B may make the sensory feedback provided by the first sensory feedback operation more easily discernible.

In some embodiments, the coordinated implementation of the first and second modalities comprises sensory feedback provided by the first sensory feedback operation being different from sensory feedback provided by the second sensory feedback operation.

The different sensory feedback provided by the first sensory feedback operation compared to that provided by the second sensory feedback operation may provide a relatively simple way of assisting the user to distinguish the feedbacks provided by the first and second pump arrangements 8A, 8B.

In some embodiments, the coordinated implementation of the first and second modalities comprises sensory feedback provided by the first sensory feedback operation having a different intensity from sensory feedback provided by the second sensory feedback operation. The term "different intensity" may mean that the sensory feedback provided by the first sensory feedback operation has a different intensity level from the intensity level of the sensory feedback provided by the second sensory feedback operation.

In some embodiments, the coordinated implementation of the first and second modalities comprises the first sensory feedback operation being asynchronous with the second expression operation. By the first sensory feedback operation being implemented at a different time from the second expression operation of the second pump arrangement 8B, the sensory feedback provided by the former may be more easily differentiated from the contribution to the sensory output provided by the latter.

In some embodiments, the coordinated implementation of the first and second modalities comprises the first sensory feedback operation being concurrent with the second sensory feedback operation. This may mean that the second sensory feedback operation may be asynchronous with the first expression operation. Thus, the sensory feedback provided by the second sensory feedback operation of the second pump arrangement 8B may be differentiated from a sensory contribution provided by the first expression operation of the first pump arrangement 8A.

In some embodiments, the first sensory feedback operation may be concurrent with, and provide identical sensory feedback to that provided by, the second sensory feedback operation.

Cooperation of the first and second controllers 10A, 10B in order to coordinate the implementation of the first and second modalities can be implemented in any suitable manner, for example the system 1 may include a wired or wireless communication link 20A, 20B connecting the first controller 10A and the second controller 10B to each other.

In some embodiments, such as that shown in FIG. 1, the system 1 comprises a communications system 20A, 20B configured to enable each of the first and second controllers to communicate with an external controller device 22 comprising a processor 24 configured to control the first and second controllers 10A, 10B to cooperate to coordinate implementation of the first and second modalities.

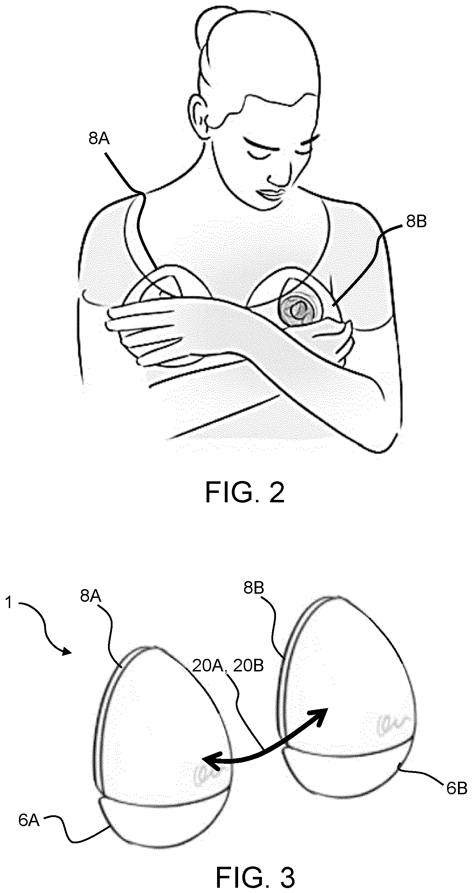

There are also wearable breast pump arrangements 8A, 8B, which are for example for mounting within a feeding bra. All of the drivetrain components described above are then formed within an outer enclosure which fits over the breast. A top part contains the drivetrain (pump 16A, 16B, motor 14A, 14B, controller 10A, 10B) and a bottom part forms the collection vessel 6A, 6B. This enables breast pumping to be performed more discretely. FIG. 2 shows a mother wearing two wearable breast pump arrangements 8A, 8B.

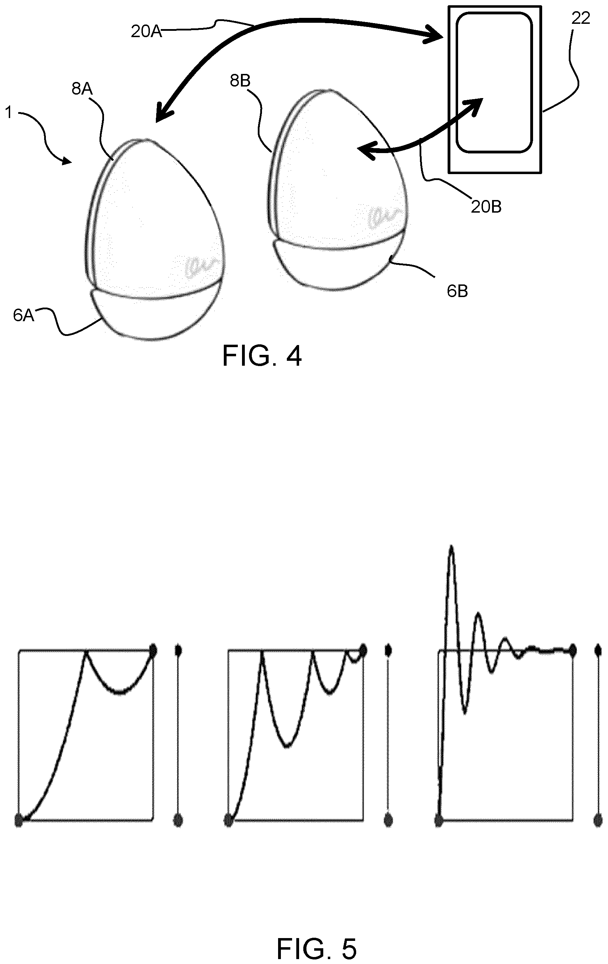

In some embodiments, such as those shown in FIGs. 3 and 4, at least one of the first pump arrangement 8A and the second pump arrangement 8B is a wearable pump arrangement.

Being assisted, via coordination of the first and second modalities, to distinguish the sensory feedback provided by the first feedback operation of the first pump arrangement from the sensory output of the second pump arrangement may be particularly valuable when one or both of the first and second pump arrangements 8A, 8B are being worn by the user.

In some embodiments, such as that shown in FIG. 3, the second controller 10B is configured to send second system data relating to the second modality to the first controller 10A, and the first controller 10A is configured to select control settings associated with the first modality based on the second system data to coordinate the first and second modalities.

Thus, the first pump arrangement 8A is not controlled as an independent unit, but its control depends on that of the second pump arrangement 8B. However, instead of a global system controller sending control commands to both pump arrangements 8A, 8B, one of the controllers 10A, 10B sends its own system data to the other, thus implementing a control loop between the two pump arrangements 8A, 8B.

The first controller 10A may also send first system data relating to the first modality to the second controller 10B. The second controller 10B may then use this first system data to select control settings associated with the second modality.

Thus, in this case, system data is shared between the first and second controllers 10A, 10B and one or both can adapt their local settings in dependence on the system data of the other.

The system data may be the settings selected by the user, such as an intensity level or a mode of operation, or it may be state information such as timing information or pressure level information. The system data may include sensor data such as pressure sensor data, motion or orientation sensor data, microphone sensor data etc.

In FIG. 3, the communications channel and hence synchronizing system is represented as a communication link 20A, 20B between the first and second pump arrangements 8A, 8B. Using this link 20A, 20B, intensity settings and timing information may be shared between the first and second controllers 10A, 10B.

The first and second pump arrangements 8A, 8B may be different from each other, but the communication link 20A, 20B may nonetheless enable such differences to be tolerated.

By sharing system data, such as intensity setting information between the first and second controllers 10A, 10B (or at least from one to the other) synchronization can take place. For example, one of the first and second controllers 10A, 10B can periodically send a notification identifying an operation and/or a timing of an operation to the other of the first and second controllers 10B, 10A. This timing information may then be part of the transferred system data.

At least one of the first and second controllers 10A, 10B has an input for receiving the delivered data (directly or via an external intermediary) to the other of the first and second controllers 10B, 10A. The received data is again the system data discussed above.

Preferably, the communication link is bidirectional so each controller 10A, 10B can send system data to, and receive system data from, the other controller 10B, 10A.

In this way, the two pump arrangements 8A, 8B are not independently controlled, either by respective local controllers 10A, 10B or by a shared system controller. Instead, the overall system control is based on communication between two local controllers 10A, 10B.

The communication link 20A, 20B is for example a Bluetooth connection directly between the first and second controllers 10A, 10B, with no communication to an external controller. One or both of the first and second controllers 10A, 10B may output status information to a remote device, but in this example the control functions are implemented by the devices themselves. A different communication technology may of course be used such IR, NFC, RF, and so on.

In some embodiments, one of the first and second controllers 10A, 10B may be a master controller with the other of the first and second controllers 10B, 10A being a slave controller. In such embodiments, communication may only be needed from the master controller to the slave controller.

In such embodiments, the first and second pump arrangements 8A, 8B may be identical so that the master-slave relationship is established by communication rather than by the fixed hardware design of the first and second pump arrangements 8A, 8B.

In this way, either of the first and second pump arrangements 8A, 8B may be used on its own, and another may be added when the two pump arrangements 8A, 8B are to be used in the synchronized way. There may instead be separate master and master and slave device designs.

FIG. 4 shows a further example of a milk expression system 1, again comprising a first wearable pump arrangement 8A and a second wearable pump arrangement 8B, each with a respective milk collection vessel 6A, 6B.

In this example, the first and second pump arrangements 8A, 8B each have a communications system 20A, 20B for communicating with an external controller device 22. For example, the external controller device 22 includes a computer program, e.g. mobile application, which is run on the external controller device 22, such as a smartphone or tablet computer. The smartphone or tablet computer can also provide a user interface, providing feedback to the user and/or receiving control instructions from the user.

FIG. 5 shows three possible feedback intensity profiles of amplitude versus time. The feedback may be audible and/or tactile.

FIG. 5 shows in schematic form that the feedback profile may have a desired pulsing frequency as well as a desired amplitude characteristic, either with sudden changes in amplitude (e.g. clicks or sharp vibrations) or gradual amplitude changes.

Thus, the feedback signal in these examples comprises a sequence of feedback pulses, induced by suitable control of the operation of the first drive arrangement, e.g. the motor 14A, pump 16A and/or valve 18A. The timing, amplitude response and the overall duration of the pulses may then encode different feedback messages.

Tactile signals may also be distinguished from each other by a similar approach, with different vibration frequency, vibration amplitude and vibration duration.

The example shown in FIG. 1 comprises a first drive arrangement which comprises the motor 14A, pump 16A and/or valve 18A (and a second drive arrangement which comprises the motor 14B, pump 16B and/or valve 18B). However, other drive arrangements are possible.

For example, an arrangement may be used to create a base vacuum which is modulated around a preferred level. Such a base vacuum can be created manually or mechanically by, for example, a servo-actuated bellows. Vacuum modulation can be created for example lowering the air pressure in a defined volume by a pump or increasing the vacuum volume by a mechanical actuation.

As another example, a drive arrangement can be made based on a plunger-based pumping system creating either a peak vacuum which is cyclically released by a valve or a base vacuum which is modulated by, for instance, the same plunger pump.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A milk expression system (1) comprising:
a first pump arrangement (8A) configured to operate according to a first modality comprising a first expression operation and a first sensory feedback operation; and
a second pump arrangement (8B) configured to operate according to a second modality comprising a second expression operation, wherein the first and second pump arrangements are configured to cooperate to coordinate implementation of the first and second modalities.

2. The milk expression system (1) according to claim 1, wherein said coordinated implementation of the first and second modalities comprises the first sensory feedback operation being provided while the second expression operation of the second pump arrangement is paused.

3. The milk expression system (1) according to claim 1 or claim 2, wherein the second modality further comprises a second sensory feedback operation.

4. The milk expression system (1) according to claim 3, wherein said coordinated implementation of the first and second modalities comprises sensory feedback provided by the first sensory feedback operation being different from sensory feedback provided by the second sensory feedback operation.

5. The milk expression system (1) according to claim 3 or claim 4, wherein said coordinated implementation of the first and second modalities comprises sensory feedback provided by the first sensory feedback operation having a different intensity, duration, and/or number of repetitions in a feedback signal relative to sensory feedback provided by the second sensory feedback operation.

6. The milk expression system (1) according to any of claims 3 to 5, wherein said coordinated implementation of the first and second modalities comprises the first sensory feedback operation being concurrent with the second sensory feedback operation.

7. The milk expression system (1) according to any of claims 1 to 6, wherein said coordinated implementation of the first and second modalities comprises the first sensory feedback operation being asynchronous with the second expression operation.

8. The milk expression system (1) according to any of claims 1 to 7, wherein the first and second pump arrangements (8A, 8B) are controllable by one or more controllers (10A, 10B) to coordinate the first and second modalities.

9. The milk expression system (1) according to any of claims 1 to 8, comprising a communications system (20A, 20B) configured to enable each of the first and second pump arrangements (8A, 8B) to communicate with an external controller device (22) configured to control the first and second pump arrangements (8A, 8B) to cooperate to coordinate implementation of the first and second modalities.

10. The milk expression system (1) according to any of claims 1 to 9, wherein the first expression operation comprises a stimulation mode and an extraction mode.

11. The milk expression system (1) according to any of claims 1 to 10, wherein the sensory feedback comprises visual, tactile and/or audible feedback.

12. The milk expression system (1) according to any of claims 1 to 11, wherein the first pump arrangement (8A) includes a first drive arrangement (14A, 16A) configured to implement the first expression operation, wherein the drive arrangement is further configured to implement the first sensory feedback operation.

13. The milk expression system (1) according to any of claims 1 to 12, comprising:
a first expression kit (2A) comprising the first pump arrangement (8A); and
a second expression kit (2B) comprising the second pump arrangement (8B); optionally wherein at least one of the first expression kit and the second expression kit is a wearable expression kit.

14. A method for operating a milk expression system (1) having a first pump arrangement (8A) and a second pump arrangement (8B), the method comprising:
controlling the first pump arrangement according to a first modality comprising a first expression operation and a first sensory feedback operation; and
controlling the second pump arrangement according to a second modality, wherein the controlling the first pump arrangement and the controlling the second pump arrangement are cooperative to coordinate implementation of the first and second modalities.

15. A computer program product comprising computer program code which is configured, when said computer program is run on one or more processors included in a milk expression system (1) further comprising a first pump arrangement (8A) and a second pump arrangement (8B), to cause the one or more processors to implement the method of claim 14.
